# EUROPÄISCHE PATENTSCHRIFT

(11) **EP 1 344 517 B1**
(45) Veröffentlichungstag und Bekanntmachung des Hinweises auf die Patenterteilung: **06.07.2011**
(21) Anmeldenummer: 03004623.9
(22) Anmeldetag: 03.03.2003
(51) Int. Cl.: A61K 8/81, A61K 8/55, A61Q 19/00

(54) **Stabile Dispersionskonzentrate**
Stable dispersion concentrates
Dispersions concentrées stables

(30) Priorität: 14.03.2002 DE 10211140
(43) Veröffentlichungstag der Anmeldung: 17.09.2003
(73) Patentinhaber: Clariant Produkte (Deutschland) GmbH, 65929 Frankfurt am Main (DE)
(72) Erfinder: Löffler, Matthias, Dr., 65527 Niedernhausen (DE); Morschhäuser, Roman, Dr., 55122 Mainz (DE); Da Rocha, Livio Caribé, CEP 04609-033, Sao Paulo (BR)

(56) Entgegenhaltungen:
- EP-A- 1 028 129
- EP-A- 1 116 733
- EP-A- 1 236 464

## Beschreibung

### Stabile Dispersionskonzentrate

Die vorliegende Erfindung betrifft Dispersionskonzentrate, enthaltend Copolymere auf Basis von Acryloyldimethyltaurinsäure bzw. deren Salze (AMPS) und linearen und/oder cyclischen N-Vinylcarbonsäureamiden.

In EP 1 116 733 und EP 1 028 129 werden neue Klassen von Polymeren auf Basis von Acryloyldimethyltaurinsäure bzw. deren Salze beschrieben. Diese decken breite anwendungstechnische Eigenschaften ab und können als Verdicker, Konsistenzgeber, Emulgator, Dispergator, Gleitmittel, Conditioner und/oder Stabilisator in kosmetischen, dermatologischen und pharmazeutischen Mitteln eingesetzt werden.
Die bevorzugt durch Fällungspolymerisation hergestellten Copolymere auf Basis von AMPS entsprechend dem Stand der Technik sind pulverförmige Substanzen mit dadurch resultierenden anwendungstechnischen Nachteilen. Neben einer Staubexplosionsgefahr kann der Staub Gefahren bei Inhalation bergen, ferner ist die Lagerstabilität der Pulver durch Hygroskopie beeinträchtigt.
Bei Verarbeitung bzw. Verwendung der pulverförmigen Produkte ist der Lösevorgang (bevorzugt werden die Polymere in wässrige Medien eingearbeitet) meistens sehr zeitaufwendig. Der Lösevorgang der pulverförmigen Produkte kann, je nach Ansatzgröße eine Stunde und mehr betragen. Zudem wird häufig eine unvollständige Lösung/Aufquellung der pulverförmigen Produkte beobachtet, was zu einer Qualitäts- und Stabilitätsverminderung der Endformulierung führt (Klumpenund Quaddelbildung). Des weiteren sind bei der Verarbeitung bzw. Verwendung der pulverförmigen Produkte besondere Rühr- und Dispergiervorrichtungen erforderlich, um die Polymere zu lösen bzw. zu suspendieren.

Zielsetzung war, flüssige Zubereitungen der pulverförmigen Polymere auf Basis von Acryloyldimethyltaurinsäure bzw. deren Salze, bevorzugt hergestellt durch Fällungspolymerisation, zu finden. Bevorzugt sind dabei Dispersionen der Polymere in einer flüssigen Matrix enthaltend Öl, Emulgator, Dispergator und/oder Wasser. Bevorzugt sind dabei flüssig-disperse Formen mit möglichst hohem Polymeranteil, niedriger Viskosität bei gleichzeitig hoher Stabilität der Dispersion. Die verwendeten Öl- und Emulgator/Dispergatoranteile sind bevorzugt kosmetische und pharmazeutisch akzeptable Rohstoffe.

Überraschenderweise wurde gefunden, dass sich AMPS-Copolymere hervorragend eignen zur Herstellung von Dispersionskonzentraten.

Gegenstand der Erfindung sind gießfähige Dispersionskonzentrate, enthaltend
I) 20 bis 80 Gew.-%, bevorzugt 20 bis 60 Gew.-%, besonders bevorzugt 30 bis 40 Gew.-% eines Copolymeren bestehend im wesentlichen aus
   a) 1 bis 50 Gew.-% der wiederkehrenden Struktureinheit der Formel (1) 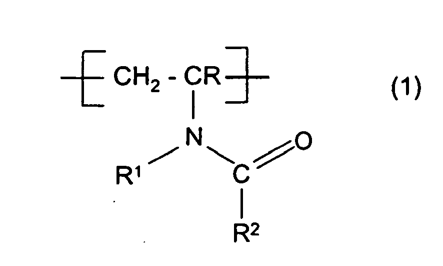 wobei R, R¹ und R² gleich oder verschieden sein können und Wasserstoff oder eine lineare oder verzweigte Alkyl- oder Alkenylgruppe mit jeweils 1 bis 30, bevorzugt 1 bis 20, insbesondere 1 bis 12 C-Atomen bedeuten, oder R¹ und R² zusammen eine C₂-C₉-Alkylengruppe bedeuten,
   b) 49,99 bis 98,99 Gew.-% der wiederkehrenden Struktureinheit der Formel (2) 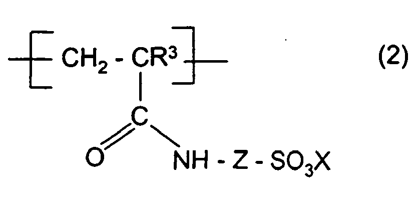 worin R³ Wasserstoff, Methyl oder Ethyl, Z C₁-C₈-Alkylen und X Ammonium-, Alkali- oder Erdalkali-Ion bedeutet, sowie
   c) 0,01 bis 8, vorzugsweise 0,01 bis 5 Gew.-% vernetzenden Strukturen, die aus Monomeren mit mindestens zwei olefinischen Doppelbindungen hervorgegangen sind,
II) 20 bis 80 Gew.-%, bevorzugt 30 bis 80 Gew.-%, besonders bevorzugt 40 bis 60 Gew.-% eines oder mehrerer Emulgatoren ausgewählt aus flüssigen Fettsäureestern, die sowohl ethoxyliert als auch nicht ethoxyliert sein können, Sorbitolestern, hergestellt durch Reaktion von Sorbitol mit Fettsäuremethylestern oder Fettsäuretriglyceriden, wobei die Sorbitolester auch alkoxyliert, vorzugsweise ethoxyliert, sein Können, und ethoxylierten und nicht ethoxylierten mono-, oli- oder tri-Phosphorsäureestern und einer Ölphase und
III) 0 bis 30 Gew.-%, bevorzugt 0 bis 10 Gew.-%, besonders bevorzugt 0 bis 5 Gew.-% Wasser.

Bevorzugt enthalten die erfindungsgemäßen Dispersionskonzentrate Copolymere, bestehend aus 2 bis 30, insbesondere 3 bis 15 Gew.-% an Struktureinheiten der allgemeinen Formel (1), vorzugsweise abgeleitet von N-Vinylpyrrolidon, 69,5 bis 97,5, insbesondere 84,5 bis 96,5 Gew.-% an Struktureinheiten der allgemeinen Formel (2), vorzugsweise abgeleitet vom Ammoniumsalz der 2-Acrylamido-2-methylpropan-sulfonsäure und 0,2 bis 3, insbesondere 0,5 bis 2 Gew.-% an vernetzenden Strukturen, die aus Monomeren mit mindestens zwei olefinischen Doppelbindungen hervorgegangen sind. Die Copolymere können auch Mischungen verschiedener Struktureinheiten innerhalb der Formel (1) enthalten, vorzugsweise Mischungen aus Monomeren mit cyclischen und offenen Carbonamid-Gruppen.
Das Mischungsverhältnis kann dabei innerhalb beliebiger Grenzen variieren.

Vernetzende Strukturen, die aus Monomeren mit mindestens zwei olefinischen Doppelbindungen hervorgegangen sind, leiten sich vorzugsweise ab von Acryl- oder Methacrylsäureallylester, Dipropylenglykol-diallylether, Polyglykoldiallylether, Triethylen-glykoldivinylether, Hydrochinon-diallylether, Tetraallyloxyethan oder anderen Allyl- oder Vinylethern multifunktioneller Alkohole, Tetraethylenglykol-diacrylat, Triallylamin, Trimethylolpropandiallylether, Methylen-bis-acrylamid oder Divinylbenzol.
Besonders bevorzugt leiten sich die vernetzenden Strukturen ab von Monomeren der allgemeinen Formel (3), 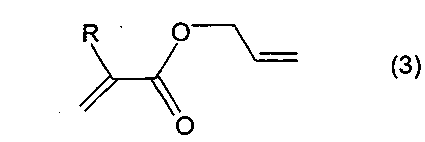 worin R Wasserstoff, Methyl oder Ethyl bedeutet.

Die Herstellung der den erfindungsgemäßen Dispersionskonzentraten zugrunde liegenden Copolymeren erfolgt wie in EP 1 116 733 und EP 1 028 129 beschrieben, indem man die den wiederkehrenden Struktureinheiten der Formeln (1) und (2) entsprechenden Monomeren in einem protischen Lösungsmittel löst oder dispergiert, zu dieser Lösung oder Dispersion einen oder mehrere Vernetzer mit mindestens zwei olefinischen Doppelbindungen zugibt und die Polymerisation in an sich bekannter Weise durch Zugabe einer radikalbildenden Verbindung startet.

Bei den Acryloyldimethyltauraten kann es sich um die anorganischen oder organischen Salze der Acryloyldimethyltaurinsäure (Acrylamidopropyl-2-methyl-2-sulfonsäure) handeln. Bevorzugt werden die Li⁺-, Na⁺-, K⁺-, Mg⁺⁺-, Ca⁺⁺-, Al⁺⁺⁺und/oder NH₄⁺-Salze.

Der Neutralisationsgrad der Acryloyldimethyltaurinsäure kann zwischen 0 und 100 % betragen, besonders bevorzugt ist ein Neutralisationsgrad von oberhalb 80 %.

Die erfindungsgemäßen Dispersionskonzentrate enthalten neben dem Copolymer noch einen oder mehrere Emulgatoren und eine Ölphase in der angegebenen Menge.

Als Emulgatoren kommen in Betracht flüssige Fettsäureester, die sowohl ethoxyliert (PEG-10 Polyglyceryl-2 Laurate) als auch nicht ethoxyliert (Polyglyceryl-2 Sesquiisostearate) sein können, oder Sorbitolester, hergestellt durch Reaktion von Sorbitol mit Fettsäuremethylestern oder Fettsäuretriglyceriden. Der Fettsäurerest in den Fettsäuremethylestern und Fettsäuretriglyceriden enthält im allgemeinen 8 bis 22 C-Atome und kann geradkettig oder verzweigt, gesättigt oder ungesättigt sein. Beispiele hierfür sind Palmitinsäure, Stearinsäure, Laurinsäure, Linolsäure, Linolensäure, Isostearinsäure oder Ölsäure. Als Fettsäuretriglyceride kommen alle nativen tierischen oder pflanzlichen Öle, Fette und Wachse in Frage, beispielsweise Olivenöl, Rapsöl, Palmkernöl, Sonnenblumenöl, Kokosöl, Leinöl, Ricinusöl, Sojabohnenöl, gegebenenfalls auch in raffinierter oder hydrierter Form. Da diese natürlichen Fette, Öle und Wachse normalerweise Mischungen von Fettsäuren mit unterschiedlicher Kettenlänge darstellen, gilt dies auch für die Fettsäurereste in den erfindungsgemäß eingesetzten Sorbitolestern. Die erfindungsgemäß eingesetzten Sorbitolester können auch alkoxyliert sein, vorzugsweise ethoxyliert.

Des weiteren können ethoxylierte und nicht ethoxylierte mono-, di- oder tri-Phosphorsäureestereingesetzt werden.

Ebenfalls geeignet sind Gemische von Verbindungen aus mehreren dieser Substanzklassen.

Die erfindungsgemäßen Dispersionen enthalten im Gemisch mit einem Emulgator ein oder mehrere Öl/e, bevorzugt aus der Gruppe der Kohlenwasserstoffe, Esteröle, pflanzlichen Öle und Silikonöle. Kohlenwasserstofföle sind zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, hydrierte Polyisobutylene, Docosane, Hexadecan, Isohexadecan, Paraffine und Isoparaffine;
Öle pflanzlichen Ursprungs sind insbesondere flüssige Triglyceride wie Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Olivenöl, Erdnussöl, Rapsöl und Kokosnussöl; Öle tierischen Ursprungs sind beispielweise Rindertalg, Perhydrosqualen, Lanolin.

Weiterhin kommen in Frage synthetische Öle wie Purcellinöl, lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Alkoholester von C₁-C₁₀-Carbonsäuren oder C₂-C₃₀-Dicarbonsäuren, C₁-C₃₀-Carbonsäuremonoester und Polyester von Zucker, C₁-C₃₀-Monoester und Polyester von Glycerin; Wachse wie Bienenwachs, Paraffinwachs oder Mikrowachse, gegebenenfalls in Kombination mit hydrophilen Wachsen, wie z.B. Cetylstearylalkohol; Fluorierte und perfluorierte Öle;
Monoglyceride von C₁-C₃₀-Carbonsäuren, Diglyceride von C₁-C₃₀-Carbonsäuren, Triglyceride von C₁-C₃₀-Carbonsäuren, beispielsweise Triglyceride der Capryl/Caprinsäuren, Ethylenglykolmonoester von C₁-C₃₀-Carbonsäuren, Ethylenglycoldiester von C₁-C₃₀-Carbonsäuren, Propylenglycolmonoester von C₁-C₃₀-Carbonsäuren, Propylenglycoldiester von C₁-C₃₀-Carbonsäuren, sowie propoxilierte und ethoxilierte Derivate der oben genannten Verbindungsklassen.

Die erfindungsgemäßen Dispersionen können zusätzlich auch 0 bis 30 Gew.-%, bevorzugt 0 bis 10 Gew.-%, besonders bevorzugt 0 bis 5 Gew.-% Wasser enthalten.

Die Herstellung der erfindungsgemäßen Dispersionskonzentrate kann auf verschiedene Weise erfolgen, wobei eine inverse Emulsionspolymerisation oder eine inverse Mini-Emulsionspolymerisation genauso bevorzugt ist wie eine physikalische Abmischung von Copolymer mit Öl und Emulgator und ggfs. Wasser. Die physikalische Abmischung erfolgt in der Weise, dass Ölphase und Emulgator/en bei 10°C bis 60°C, bevorzugt bei Raumtemperatur vermischt werden, zu ca. 40 Gew.-% der ÖI-/Emulgatorphase Copolymere in einem Zeitraum von 10 bis 60 Min., bevorzugt ca. 30 Min. unter kräftigem Rühren zugegeben wird. Dabei bildet sich eine homogene Paste. Wenn erforderlich, kann eine geringe Menge an Wasser zum besseren Verarbeiten hinzugefügt werden. Anschließend wird die restliche ÖI-/ Emulgatorphase unter Rühren zugegeben und mehrere Stunden homogen verrührt. Es resultiert eine flüssige, gießfähige Dispersion.

Die erfindungsgemäßen Dispersionskonzentrate eignen sich als Verdicker, Konsistenzgeber, Emulgator, Solubilisator, Dispergator, Gleitmittel, Haftmittel, Conditioner und/oder Stabilisator - hervorragend zur Formulierung von kosmetischen, pharmazeutischen und dermatologischen Mitteln, insbesondere von Öl-in-Wasser Emulsionen in Form von Cremes, Lotionen, Reinigungsmilch, Cremegele, Sprühemulsionen, z.B. Bodylotions, After-Sun Lotions, Sonnenschutzmittel und Deo-Sprays,

Der Vorteil dieser Dispersionskonzentrate liegt darin, dass hier die oben definierten Copolymere in einer Darreichungsform vorliegen, die eine leichte Herstellung von pharmazeutischen und kosmetischen Zubereitungen auf der Basis dieser Copolymere ermöglicht. Die erfindungsgemäßen Dispersionskonzentrate sind überraschenderweise trotz ihres hohen Anteils an Copolymer gießfähig und lagerstabil.

Die erfindungsgemäßen Dispersionskonzentraten werden in den kosmetischen und pharmazeutischen Zubereitungen in solchen Gewichtsmengen eingesetzt, dass Polymerkonzentrationen von 0,01 bis 10 Gew.-%, bevorzugt 0,1 bis 5 Gew.-%, besonders bevorzugt 0,5 bis 3 Gew.-%, bezogen auf die fertigen Mittel resultieren.

Solche Zubereitungen können anionische, kationische, nichtionische, zwitterionische und/oder amphotere Tenside, sowie weitere Hilfs- und Zusatzstoffe, kationische Polymere, Filmbildner, Überfettungsmittel, Stabilisatoren, biogene Wirkstoffe, Glycerin, Konservierungsmittel, Perlglanzmittel, Farb- und Duftstoffe, Lösungsmittel, Trübungsmittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen Mitteln antimikrobiell wirkende Agentien zugesetzt werden.

Zusätzlich können derartige organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol. Hydrotrop wirken kurzkettige Aniontenside, insbesondere Arylsulfonate, beispielsweise Cumol- oder Toluolsulfonat.

Die nachfolgenden Beispiele von Dispersionskonzentraten mit AMPS Polymeren sollen den Gegenstand der Erfindung näher erläutern, ohne ihn darauf einzuschränken (bei den Prozentangaben handelt es sich um Gew.-%).

Es wurden verschiedene Dispersionskonzentrate mit unterschiedlichen Emulgatorund Ölkonzentration hergestellt. Dabei wurden ® Aristoflex AVC sowie ® Aristoflex AVC-1 (Clariant) eingesetzt.

Tabelle 1 zeigt Beispiele von Dispersionskonzentraten, die fließfähig und lagerstabil (Sedimentierung bei Lagerung 25°C 3 Wochen) sind.

| | | A | B | C | D | E | F | G | H |
|---|---|---|---|---|---|---|---|---|---|
| 1 | Aristoflex AVC | 36 | 36 | 36 | 30 | | | | |
| 2 | Aristoflex AVC-1 | | | | | 36 | 36 | 36 | 30 |
| 3 | Hostacerin DGI | | 30 | 3 | 51 | | 30 | 3 | 51 |
| 4 | Hostaphat KL 340 D | 18 | 18 | 2 | 13 | 18 | 18 | 2 | 13 |
| 5 | Emulsogen SRO | 30 | | | | 30 | | | |
| 6 | Mineral Öl, niedrig viskos | 8 | | 29,5 | 6 | 8 | | 29,5 | 6 |
| 7 | Isopropyl Palmitate | 8 | | 29,5 | | 8 | | 29,5 | |
| 8 | Myritol 318 | | 16 | | | | 16 | | |

Die Herstellung dieser Dispersionskonzentrate erfolgte wie folgt:
1. Öl- und Emulgatorkomponente 3-8 mischen und ein Drittel der Mischung vorlegen.
2. In einer halben Stunde Polymer 1-2 unter Rühren bei 400 upm zugeben.
3. Eine halbe Stunde nachrühren dann die restliche Mischung der Öl- und Emulgatorkomponente zugeben.
4. Weitere 5 Stunden nachrühren.

### Struktur der benutzten Handelsprodukte:

| | | INCI-Name |
|---|---|---|
| 1 | Aristoflex AVC | Ammonium Acryloyldimetyltaurate/VP Copolymer |
| 2 | Aristoflex AVC-1 | Ammonium Acryloyldimethyltaurate/Vinylformamide Copolymer |
| 3 | Hostacerin DGI | Polyglyceryl-2-Sesquiisostearate |
| 4 | Hostaphat KL 340 D | Trilaureth-4 Phosphate |
| 5 | Emulsogen SRO | Rapeseed Oil Sorbitol Esters |
| 6 | | Mineral Öl, niedrig viskos |
| 7 | | Isopropyl Palmitate |
| 8 | Myritol 318 | Caprylic/Capric Triglyceride |

Beispiele zur Verwendung der erfindungsgemäßen Dispersionskonzentrate bei der Herstellung kosmetischer Präparate.

### Beispiel 1: Feuchtigkeitsspendende Lotion

| | | |
|---|---|---|
| A | Almondöl | 7,00 % |
| | Cyclomethicone | 5,00 % |
| B | Dispersionskonzentrat C | 4,00 % |
| C | Glycerin | 7,00 % |
| | Water | ad 100% |
| | Konservierungsmittel | q.s. |
| D | Duftstoff | 0,30 % |

| Herstellung | |
|---|---|
| I | A und B mischen. |
| II | Lösung von C in I einrühren. |
| III | D zu II geben. |
| IV | homogenisieren |
| V | pH 5,5 |

### Beispiel 2: Sonnenschutzlotion

| | | |
|---|---|---|
| A | Vaselin | 5,00 % |
| | Paraffinöl | 10,00 % |
| | Dispersionskonzentrat A | 2,00 % |
| | Tocopherylacetat | 1,00 % |
| | Octylmethoxycinnamat | 2,00 % |
| | Parsol 1789 | 0,20 % |
| B | Ethanol | 10,00 % |
| C | Butylenglycol | 5,00 % |
| | Wasser | ad 100 % |

| Herstellung | |
|---|---|
| I | A und C werden getrennt auf 75°C erwärmt, danach vereinigt und unter Rühren auf 65°C abgekühlt, homogenisiert und weiter auf 35°C abgekühlt, |
| II | B in l einrühren, homogenisieren und auf Raumtemperatur abkühlen |

### Beispiel 3: O/W - Hautmilch

| Zusammensetzung | | |
|---|---|---|
| A | Isopropylpalmitat | 4,00 % |
| | Mandelöl 5,00 % | 4,00 % |
| | Weizenkeimöl | 1,00 % |
| | ® Cetiol SN (Henkel) | 8,00 % |
| | Cetearylisononanoat | |
| B | Dispersionskonzentrat G | 1,50 % |
| C | Wasser | ad 100 % |
| D | Duftstoffe | 0,30 % |

| Herstellung | |
|---|---|
| I | B unter Rühren zu A hinzugeben |
| II | C und D zu l hinzurühren |
| III | Emulsion homogenisieren |

## Patentansprüche

1. Gießfähige Dispersionskonzentrate, enthaltend:
I) 20 bis 80 Gew,-% eines Copolymeren bestehend im Wesentlichen aus
a) 1 bis 50 Gew.-% der wiederkehrenden Struktureinheit der Formel (1) 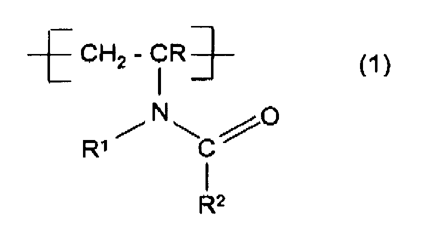 wobei R, R¹ und R² gleich oder verschieden sein können und Wasserstoff oder eine lineare oder verzweigte Alkyl- oder Alkenylgruppe mit jeweils 1 bis 30, bevorzugt 1 bis 20, insbesondere 1 bis 12 C-Atomen bedeuten, oder R¹ und R² zusammen eine C₂-C₉-Alkylengruppe bedeuten,
b) 49,99 bis 98,99 Gew.-% der wiederkehrenden Struktureinheit der Formel (2) 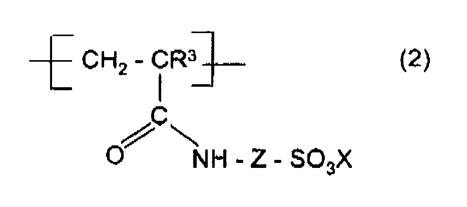 worin R³ Wasserstoff, Methyl oder Ethyl, Z C₁-C₈-Alkylen und X Ammonium-, Alkali- oder Erdalkali-Ion bedeutet, sowie
c) 0,01 bis 8, vorzugsweise 0,01 bis 5 Gew.-% vernetzenden Strukturen, die aus Monomeren mit mindestens zwei olefinischen Doppelbindungen hervorgegangen sind,
II) 20 bis 80 Gew.-% eines oder mehrerer Emulgatoren ausgewählt aus flüssigen Fettsäureestern, die sowohl ethoxyliert als auch nicht ethoxyliert sein können, Sorbitolestern, hergestellt durch Reaktion von Sorbitol mit Fettsäuremethylestem oder Fettsäuretriglyceriden, wobei die Sorbitolester auch alkoxyliert, vorzugsweise ethoxyliert, sein können, und ethoxylierten und nicht ethoxylierten mono-, di- oder tri-Phosphorsäureestern und einer Ölphase, sowie
III) 0 bis 30 Gew.-% Wasser.

2. Dispersionskonzentrat gemäß Anspruch 1, **dadurch gekennzeichnet, dass** das Copolymer aus 2 bis 30, insbesondere 3 bis 15 Gew.-% an Struktureinheiten der allgemeinen Formel (1), vorzugsweise abgeleitet von N-Vinylpyrrolidon, 69,5 bis 97,5, insbesondere 84,5 bis 96,5 Gew.-% an Struktureinheiten der allgemeinen Formel (2), vorzugsweise abgeleitet vom Ammoniumsalz der 2-Acrylamido-2-methyl-propan-sulfonsäure und 0,2 bis 3, insbesondere 0,5 bis 2 Gew.-% an vernetzenden Strukturen, die aus Monomeren mit mindestens zwei olefinischen Doppelbindungen hervorgegangen sind, besteht.

3. Dispersionskonzentrat gemäß Anspruch 1, **dadurch gekennzeichnet, dass** das Copolymer vernetzende Strukturen aufweist, die aus Monomeren mit mindestens zwei olefinischen Doppelbindungen hervorgegangen sind und sich vorzugsweise ableiten von Acryl- oder Methacrylsäureallylester, Dipropylenglykol-diallylether, Polyglykoldiallylether, Triethylen-glykoldivinylether, Hydrochinon-diallylether, Tetraallyloxyethan oder anderen Allyl- oder Vinylethern multifunktioneller Alkohole, Tetraethylenglykol-diacrylat, Triallylamin, Trimethylolpropandiallylether, Methylenbis-acrylamid oder Divinylbenzol.

4. Dispersionskonzentrat gemäß Anspruch 1, **dadurch gekennzeichnet, dass** das Copolymer vernetzende Strukturen aufweist, die sich aus Monomeren der allgemeinen Formel (3) ableiten, 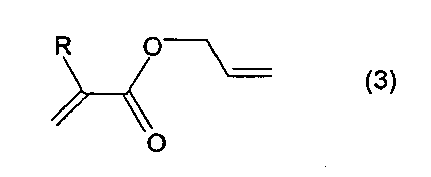 worin R Wasserstoff, Methyl oder Ethyl bedeutet.

5. Dispersionskonzentrat nach Anspruch 1, **dadurch gekennzeichnet, dass** es 20 bis 60 Gew.-% des Copolymers enthält.

6. Dispersionskonzentrat nach Anspruch 1, **dadurch gekennzeichnet, dass** es 30 bis 40 Gew.-% des Copolymers enthält.

7. Dispersionskonzentrat nach Anspruch 1, **dadurch gekennzeichnet, dass** es 30 bis 80 Gew.-% Emulgator und Ölphase enthält.

8. Dispersionskonzentrat nach Anspruch 1, **dadurch gekennzeichnet, dass** es 40 bis 60 Gew.-% Emulgator und Ölphase enthält.

9. Dispersionskonzentrat nach Anspruch 1, **dadurch gekennzeichnet, dass** es 0 bis 10 Gew.-% Wasser enthält.

10. Dispersionskonzentrat nach Anspruch 1, **dadurch gekennzeichnet, dass** es 0 bis 5 Gew.-% Wasser enthält.

11. Dispersionskonzentrat nach Anspruch 1, **dadurch gekennzeichnet, dass** die Ölphase ausgewählt ist aus einem oder mehreren Ölen der Gruppe der Kohlenwasserstoffe, Esteröle, pflanzlichen Öle und Silikonöle.

12. Verwendung der Dispersionskonzentrate nach Anspruch 1 zur Herstellung von kosmetischen, pharmazeutischen und dermatologischen Zubereitungen.

## Claims

1. A pourable dispersion concentrate comprising:
I) 20 to 80% by weight of a copolymer consisting essentially of
a) 1 to 50% by weight of the repeat structural unit of the formula (1) 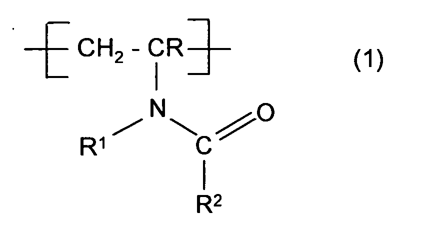 where R, R¹ and R² may be identical or different and are hydrogen or a linear or branched alkyl or alkenyl group having in each case 1 to 30, preferably 1 to 20, in particular 1 to 12, carbon atoms, or R¹ and R² together are a C₂-C₉-alkylene group,
b) 49.99 to 98.99% by weight of the repeat structural unit of the formula (2) 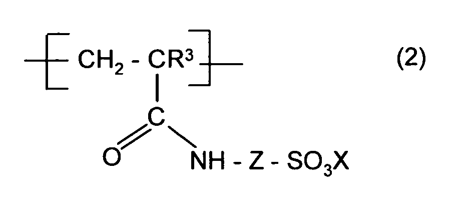 in which R³ is hydrogen, methyl or ethyl, Z is C₁-C₈-alkylene and X is ammonium, alkali metal or alkaline earth metal ion, and
c) 0.01 to 8% by weight, preferably 0.01 to 5% by weight, of crosslinking structures originating from monomers with at least two olefinic double bonds,
II) 20 to 80% by weight of one or more emulsifiers selected from liquid fatty acid esters which may either be ethoxylated or else nonethoxylated, sorbitol esters prepared by reacting sorbitol with fatty acid methyl esters or fatty acid triglycerides, where the sorbitol esters can also be alkoxylated, preferably ethoxylated, and ethoxylated and nonethoxylated mono-, di- or triphosphoric esters, and an oil phase, and
III) 0 to 30% by weight of water.

2. The dispersion concentrate as claimed in claim 1, wherein the copolymer consists of 2 to 30% by weight, in particular 3 to 15% by weight, of structural units of the formula (1), preferably derived from N-vinylpyrrolidone, 69.5 to 97.5% by weight, in particular 84.5 to 96.5% by weight, of structural units of the formula (2), preferably derived from the ammonium salt of 2-acrylamido-2-methylpropanesulfonic acid, and 0.2 to 3% by weight, in particular 0.5 to 2% by weight, of crosslinking structures originating from monomers with at least two olefinic double bonds.

3. The dispersion concentrate as claimed in claim 1, wherein the copolymer has crosslinking structures originating from monomers with at least two olefinic double bonds and preferably deriving from acrylic or methacrylic allyl esters, dipropylene glycol diallyl ether, polyglycol diallyl ether, triethylene glycol divinyl ether, hydroquinone diallyl ether, tetraallyloxyethane or other allyl or vinyl ethers of multifunctional alcohols, tetraethylene glycol diacrylate, triallylamine, trimethylolpropane diallyl ether, methylenebisacrylamide or divinylbenzene.

4. The dispersion concentrate as claimed in claim 1, wherein the copolymer has crosslinking structures derived from monomers of the formula (3), 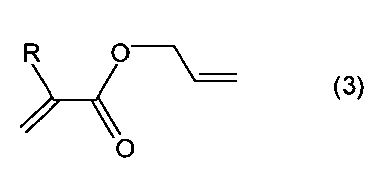 in which R is hydrogen, methyl or ethyl.

5. The dispersion concentrate as claimed in claim 1, which comprises 20 to 60% by weight of the copolymer.

6. The dispersion concentrate as claimed in claim 1, which comprises 30 to 40% by weight of the copolymer.

7. The dispersion concentrate as claimed in claim 1, which comprises 30 to 80% by weight of emulsifier and oil phase.

8. The dispersion concentrate as claimed in claim 1, which comprises 40 to 60% by weight of emulsifier and oil phase.

9. The dispersion concentrate as claimed in claim 1, which comprises 0 to 10% by weight of water.

10. The dispersion concentrate as claimed in claim 1, which comprises 0 to 5% by weight of water.

11. The dispersion concentrate as claimed in claim 1, wherein the oil phase is selected from one or more oils of the group of hydrocarbons, ester oils, vegetable oils and silicone oils.

12. The use of the dispersion concentrates as claimed in claim 1 for the preparation of cosmetic, pharmaceutical and dermatological preparations.

## Revendications

1. Concentrés de dispersions coulables, contenant :
I) 20 à 80 % en poids d'un copolymère essentiellement constitué de
a) 1 à 50 % en poids du motif répétitif de formule (1) 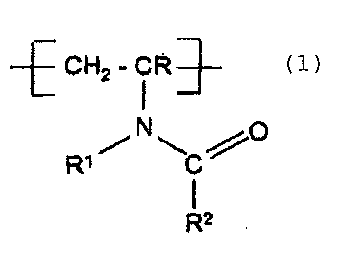 dans laquelle R, R¹ et R² peuvent être identiques ou différents et représentent un atome d'hydrogène ou un groupe alkyle ou alcényle linéaire ou ramifié ayant chacun de 1 à 30, de préférence de 1 à 20, en particulier de 1 à 12 atomes de carbone, ou R¹ et R² représentent ensemble un groupe alkylène en C₂-C₉,
b) 49,99 à 98,99 % en poids du motif répétitif de formule (2) 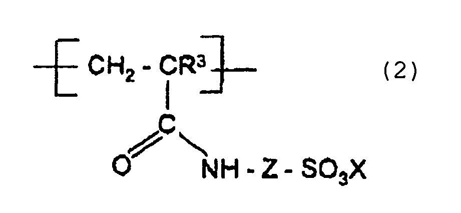 dans laquelle R³ représente un atome d'hydrogène, le groupe méthyle ou éthyle, Z représente un groupe alkylène en C₁-C₈ et X représente un ion ammonium, alcalin ou alcalino-terreux, ainsi que
c) 0,01 à 8, de préférence 0,01 à 5 % en poids de structures réticulables qui sont issues de monomères à au moins deux doubles liaisons oléfiniques,
II) 20 à 80 % en poids d'un ou plusieurs émulsifiants choisis parmi des esters liquides d'acides gras, qui peuvent être aussi bien éthoxylés que non éthoxylés, des esters de sorbitol, préparés par réaction du sorbitol avec des esters méthyliques d'acides gras ou des triglycérides d'acides gras, les esters de sorbitol pouvant également être alcoxylés, de préférence éthoxylés, et des mono-, di- ou triesters d'acide phosphorique, éthoxylés ou non éthoxylés
et d'une phase huileuse, ainsi que
III) 0 à 30 % d'eau.

2. Concentré de dispersion selon la revendication 1, **caractérisé en ce que** le copolymère est constitué de 2 à 30, en particulier 3 à 15 % en poids de motifs de formule générale (1), de préférence dérivés de N-vinylpyrrolidone, de 69,5 à 97,5, en particulier 84,5 à 96,5 % en poids de motifs de formule générale (2), de préférence dérivés de sel d'ammonium de l'acide 2-acrylamido-2-méthyl-propanesulfonique et de 0,2 à 3, en particulier 0,5 à 2 % en poids de structures réticulables qui sont issues de monomères à au moins deux doubles liaisons oléfiniques.

3. Concentré de dispersion selon la revendication 1, **caractérisé en ce que** le copolymère comporte des structures réticulables qui sont issues de monomères à au moins deux doubles liaisons oléfiniques et dérivent de préférence d'acrylate ou de méthacrylate d'allyle, d'éther diallylique de dipropylèneglycol, d'éther diallylique de polyglycol, d'éther divinylique de triéthylèneglycol, d'éther diallylique d'hydroquinone, de tétraallyloxyéthane ou d'autres éthers allyliques ou vinyliques d'alcools multifonctionnels, de diacrylate de tétraéthylèneglycol, de triallylamine, d'éther diallylique de triméthylolpropane, de méthylène-bis-acrylamide ou de divinylbenzène.

4. Concentré de dispersion selon la revendication 1, **caractérisé en ce que** le copolymère comporte des structures réticulables qui dérivent de monomères de formule générale (3) 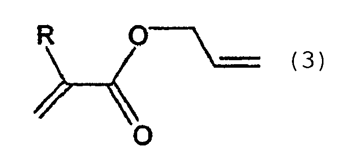 dans laquelle R représente un atome d'hydrogène, le groupe méthyle ou éthyle.

5. Concentré de dispersion selon la revendication 1, **caractérisé en ce qu'**il contient de 20 à 60 % en poids du copolymère.

6. Concentré de dispersion selon la revendication 1, **caractérisé en ce qu'**il contient de 30 à 40 % en poids du copolymère.

7. Concentré de dispersion selon la revendication 1, **caractérisé en ce qu'**il contient de 30 à 80 % en poids d'émulsifiant et de phase huileuse.

8. Concentré de dispersion selon la revendication 1, **caractérisé en ce qu'**il contient de 40 à 60 % en poids d'émulsifiant et de phase huileuse.

9. Concentré de dispersion selon la revendication 1, **caractérisé en ce qu'**il contient de 0 à 10 % en poids d'eau.

10. Concentré de dispersion selon la revendication 1, **caractérisé en ce qu'**il contient de 0 à 5 % en poids d'eau.

11. Concentré de dispersion selon la revendication 1, **caractérisé en ce que** la phase huileuse est choisie parmi une ou plusieurs huiles du groupe des hydrocarbures, huiles d'esters, huiles végétales et huiles de silicone.

12. Utilisation des concentrés de dispersions selon la revendication 1, pour la production de préparations cosmétiques, pharmaceutiques et dermatologiques.
